# EUROPEAN PATENT SPECIFICATION

(11) **EP 0 868 167 B1**
(45) Date of publication and mention of the grant of the patent: **15.05.2002**
(21) Application number: 96944033.8
(22) Date of filing: 13.12.1996
(51) Int. Cl.: A61K 7/16

(54) **DESENSITISING TOOTHPASTE**
DESENSIBILISIERUNGSZAHNPASTA
DENTIFRICE DESENSIBILISANT

(30) Priority: 20.12.1995 EP 95309274
(43) Date of publication of application: 07.10.1998
(73) Proprietor: UNILEVER N.V., 3013 AL Rotterdam (NL); UNILEVER PLC, London EC4P 4BQ (GB)
(72) Inventor: HUGHES, Nigel, Patrick, Unilever Research, Bebington Wirral, Merseyside L63 3JW (GB); LITTLEWOOD, David, L., Unilever Research, Bebington Wirral, Merseyside L63 3JW (GB); STEAD, William, John, Unilever Research, Bebington Wirral, Merseyside L63 3JW (GB)
(74) Representative: van Gent, Jan Paulus
(86) International application number: EP9605740
(87) International publication number: WO9722331

(56) References cited:
- EP-A- 0 161 898
- EP-A- 0 429 224
- EP-A- 0 512 270
- EP-A- 0 550 960

## Description

The present invention relates to the use of an amphiphilic organic material, capable of forming, upon contact with moisture, a water-insoluble liquid crystal phase of at least one dimensional periodicity, as an anti-sensitive teeth agent in the manufacture of a toothpaste for sensitive teeth.

Dentinal (hyper)sensitivity is a well-known phenomenon; people suffering from dentinal (hyper)sensitivity experience a painful reaction when their teeth are exposed to cold, hot, sweet, drying, tactile and similar stimuli, e.g. when eating ice-cream or brushing their teeth. The painful stimulus is generally attributed to rapid fluid movements within the dentinal tubules which run through the thickness of the dentine to the nerve terminals in the pre-dentine.

Many proposals have been made in the art to reduce dentinal (hyper)sensitivity, by either reducing the sensitivity of the dentinal nerves, or by blocking the dentinal tubules. The former approach involves e.g. the addition of particular salts such as potassium nitrate, potassium (bi)carbonate, potassium chloride, potassium citrate, potassium oxalate, and toothpastes with such potassium salts have found their way to the market place.

The latter approach involves, e.g. the use of strontium salts such as strontium chloride or strontium acetate or the application of a coating of polymeric materials onto the teeth, e.g. a varnish or a resin, usually done by the dentist.

Despite these prior proposals, there is still a need for an effective, improved toothpaste for alleviating dentinal (hyper)sensitivity. We have now surprisingly found, that the inclusion of an amphiphilic organic material, capable of forming, upon contact with moisture, a water-insoluble liquid crystal phase of at least one dimensional periodicity, in a toothpaste provides for a toothpaste which can significantly reduce dentinal (hyper)sensitivity. The amphiphilic organic material as defined above has been found to have dentinal (hyper)sensitivity reducing properties, or, in other words to act as an anti-sensitive teeth agent.

Amphiphilic organic material as herein defined is an organic material which has both hydrophobic and hydrophilic portions in its structure.

When water and certain water-insoluble or sparingly watersoluble organic materials are brought together, the organic materials pass through various physical phases upon the addition of water. They can form, in their final state in the aqueous medium liquid crystal structures of various dimensional periodicities. This is more fully described e.g. in *"Biological Membranes"* by D. Chapman, Academic Press New York, 1968, Chapter 3, and in "*Nature*", Vol. 222, page 1159 (1969) by Balmbra et al..

The amphiphilic organic materials according to the present invention must be capable of forming, upon addition of water or moisture (e.g. saliva), in their final state a liquid crystal phase of at least one-dimensional periodicity, e.g. a lamellar phase. Preferably, they should be capable of so forming a liquid crystal phase with a two-dimensional periodicity, e.g. a hexagonal phase, and particularly preferably they should be capable of so forming a liquid crystal phase with a three-dimensional periodicity, e.g. a cubic phase.

The use of such amphiphilic materials for certain applications has already been described in the art. Thus, in WO-A-84/02076 (Fluid-Carbon International AB), controlled-release compositions for biologically-active materials are described, comprising amphiphilic organic materials, capable of forming liquid crystalline phases, in which the biologically-active material is either dissolved or dispersed, or is coated by the liquid crystalline phase. These compositions are stated to be suitable for controlled release of medication and drug delivery.

In *"Pharmaceutical Technology Europe"* February 1995, pages 14-17, Engström et al. describe similar systems for drug delivery through the oral mucosa, exemplified by the glyceryl monooleate-water system.

In EP-A-550,960 (Unilever), amphiphilic materials capable of forming a liquid crystal phase of greater than one-dimensional periodicity are described as novel antiperspirant active materials.

None of these references are concerned with compositions for the care of the human teeth, nor do they relate to the problem of alleviating dentinal (hyper)sensitivity.

The amphiphilic organic material, suitable for use in the present invention can be selected from a host of materials, known in the art, e.g. as described in the above-mentioned references. They should, of course, be compatible with the other components of a toothpaste, and should meet the requirements of safety, taste, colour etc. that are usually set for ingredients of a toothpaste.

Typical suitable examples are unsaturated and/or saturated C₁₂-C₂₄ fatty acid glycerides, optionally in admixture with long chain fatty acids and/or fatty alcohol and/or polyalkylene glycols such as glyceryl monooleate, optionally in admixture with oleic acid, glyceryl monolaurate, in admixture with oleic acid or with oleyl alcohol, stearyl alcohol, isostearyl alcohol or a mixture thereof; glyceryl mono-isostearate, glyceryl mono-linoleate in admixture with glyceryl mono-oleate, polyoxyethylene ethers, mixtures of lecithin and oleic acid or oleyl alcohol, mixtures of sodium or potassium oleate with oleic acid or oleyl alcohol, certain silicone materials such as sodium 10-Ω-butyl [poly(dimethylsiloxy) dimethyl silyl] decanoate. Mixtures of any of these materials may also be used.

The preferred amphiphilic materials are the lipid substances, i.e. the fatty acid glycerides, in particular glyceryl monooleate, glycerylmonolinoleate, glyceryl monoisostearate, optionally in admixture with a fatty acid or fatty alcohol, and the particularly preferred material is glyceryl monooleate (GMO). In this respect it is observed, that in EP-A-429,224 a composition, suitable for insertion into or around the periodontal pocket of a person suffering from diseases of the oral cavity is described, which composition comprises monoolein (= GMO) and a drug active. The drug active can be selected from a great variety of different drug actives, among which dentinal desensitising agents such as strontium chloride or sodium fluoride are mentioned. The GMO according to this reference absorbs water from the body fluid surrounding the periodontal cavity and becomes more viscous, enabling extended duration of retention at the site of treatment, releasing the drug active in a controlled manner slowly over extended duration.

The GMO is used in this reference as a gelling agent, together with a drug active, to provide for a slow release of the drug active at the site of the treatment. This reference does not disclose that GMO is capable of reducing dentinal (hyper)sensitivity; where this reference refers to dentinal sensitivity it is in the context of adding a dentinal desensitizing agent to the GMO.

Consequently, the present invention relates to the use of amphiphilic organic materials, capable of forming upon addition of water in their final state a liquid crystal phase of at least one-dimensional periodicity as a dentinal desensitising agent in the manufacture of a toothpaste for alleviating dentinal (hyper)sensitivity.

The amphiphilic agent is generally used in the toothpaste in an amount of between 1 and 30 % by weight, preferably between 5 and 25 % by weight and particularly preferably between 5 and 15 % by weight of the toothpaste.

The toothpastes of the present invention may furthermore comprise optional, conventional ingredients such as pharmaceutically acceptable carriers like starch, sucrose, water or water/alcohol systems etc.. Small amounts of surfactants may also be included, such as anionic, nonionic, cationic and zwitterionic or amphoteric surfactants. They may comprise particulate abrasive materials such as silicas, aluminas, calcium carbonates, dicalciumphosphates, calcium pyrophosphates, hydroxyapatites, trimetaphosphates, insoluble hexametaphosphates and so on, including agglomerated particulate abrasive materials, usually in amounts between 3 and 60% by weight.

Furthermore, they may comprise humectants such as glycerol, sorbitol, propyleneglycol, xylitol, lactitol and so on.

Binders and thickeners such as sodium carboxymethylcellulose, xanthan gum, gum arabic etc. may also be included, as well as synthetic polymers such as polyacrylates and carboxyvinyl polymers such as Carbopol®.

Flavours such as peppermint and spearmint oils may also be included, as well as preservatives, opacifying agents, colouring agents, pH-adjusting agents, sweetening agents and so on.

Anti-bacterial agents may also be included such as Triclosan, chlorhexidine, copper-, zinc- and stannous salts such as zinc citrate, sodium zinc citrate and stannous pyrophosphate, sanguinarine extract, metronidazole. Further examples of anti-bacterial agents are quaternary ammonium compounds such as cetylpyridinium chloride; bis-guanides such as chlorhexidine digluconate, hexetidine, octenidine, alexidine; halogenated bisphenolic compounds such as 2,2' methylenebis-(4-chloro-6-bromophenol).

Polymeric compounds which can enhance the delivery of active ingredients such as anti-bacterial agents can also be included. Examples of such polymers are copolymers of polyvinylmethylether with maleic anhydride and other similar delivery enhancing polymers, e.g. those described in DE-A-3,942,643 (Colgate)

Furthermore anti-inflammatory agents such as ibuprofen, flurbiprofen, aspirin, indomethacin etc. may also be included.

Anti-caries agents such as sodium- and stannous fluoride, aminefluorides, sodium monofluorophosphate, casein, plaque buffers such as urea, calcium lactate, calcium glycerophosphate, strontium polyacrylates may also be included. Other optional ingredients include vitamins such as Vitamin C, and plant extracts. Additional desensitising agents such as potassium citrate, potassium chloride, potassium tartrate, potassium bicarbonate, potassium oxalate, potassium nitrate as well as strontium salts may also be included.

Buffers and salts to buffer the pH and ionic strength of the compositions may also be included. Liposomes and other encapsulates may also be used to improve delivery or stability of active ingredients.

Furthermore, the oral compositions may comprise anti-calculus agents such as alkalimetal pyrophosphates, hypophosphite-containing polymers, organic phosphonates, phosphocitrates etc..

In addition, the compositions may comprise functional biomolecules such as bacteriocins, antibodies, enzymes and so on.

Other optional ingredients that may be included are e.g. bleaching agents such as peroxy compounds e.g. potassium peroxydiphosphate, effervescing systems such as sodium bicarbonate/citric acid systems, colour change systems, and so on.

The toothpastes may also be formulated into systems for use in dual-compartment type dispensers.

The toothpastes can be manufactured in any conventional manner; when using e.g. GMO this can be added as particles to a toothpaste base by simply mixing the particles in, either as the last step in the manufacture, or as penultimate step before the flavour is added. It can also be added in another form.

The present invention will be further illustrated by way of Example.

### Example 1

A panel of subjects, suffering from dentinal sensitivitiy, was recruited. The sensitivity of each subject's teeth was measured at a baseline examination. The tooth being assessed was stimulated by a gentle air flow (about 1 litre per minute), which was initiated by a foot control by the assessor and could be stopped by the subject pressing a button when the pain became unpleasantly noticeable. The time taken to stop the flow was used as a measure of the tooth's sensitivity. Teeth, where the subject did not respond within about 10 seconds, were excluded. Following the measurement of the baseline sensitivity, the teeth were isolated using a cotton wool roll and the teeth selected at random for the application of the test and control treatment. The teeth selected for the control were 'painted' with potable water and to those for the test GMO was applied. Subjects were reassessed after a predetermined time interval, viz approximately 15 minutes. The data collected during the studies were recorded by a scribe directly on specially designed charts. The data were later entered into a computer file for statistical analysis. The data from the studies were analyzed by comparing the mean log response times for the test and control products using two-way analysis of covariance incorporating subject and treatment as factors with the initial baseline value as a covariate. The ratio of the difference in the mean adjusted response time for final reading of the study for the test and control was used as the measure of the efficacy of a treatment. GMO was applied either in lamellar form (93 % GMO/7 % water) or in cubic form (75 % GMO/25 % water). In both cases the water contained 0.3 % sodium benzoate as preservative.

The results from the studies are summarised in Table 1.

**Table 1**

| **GMO-form** | **Number of subjects (teeth)** | **Adjusted Mean Log10 Response Times** | | **Ratio of test to control** | **Statistical assessment** |
|---|---|---|---|---|---|
| | | GMO treatment | Water Control | | |
| Lamellar | 8 (30) | 0.944 | 0.139 | 6.4 | 0.0496 |
| Lamellar | 12 (40) | 1.220 | 0.448 | 5.9 | 0.0027 |
| Lamellar | 8 (30) | 0.890 | 0.218 | 4.7 | 0.0474 |
| Cubic | 14 (46) | 1.043 | 0.554 | 3.1 | 0.0022 |
| Cubic | 8 (29) | 0.952 | -0.237 | 15.5 | 0.0032 |

The results from all five studies showed statistically significant reduction in sensitivity for GMO relative to the water control despite the low numbers of subjects (and teeth) involved in several of the studies. These data provided clear evidence that GMO is capable of reducing dentinal hypersensitivity over a period of at least 15 minutes.

### Example 2

The following toothpaste formulation represents a formulation according to the invention, suitable for use of alleviating dentinal (hyper)sensitivity by the inclusion of GMO.

| | Mass % |
|---|---|
| Sorbitol syrup (70%) | 40.50 |
| Abrasive silica | 9.00 |
| Thickening silica | 7.20 |
| Polyethylene glycol (MW 1,500) | 4.50 |
| Sodium laurylsulphate | 1.35 |
| Titanium dioxide | 0.90 |
| Flavour oil | 0.90 |
| Sodium carboxymethylcellulose | 0.81 |
| Sodium fluoride | 0.32 |
| Potassium sorbate | 0.27 |
| Sodium saccharine | 0.15 |
| Monosodium phosphate | 0.09 |
| GMO (post-dosed in particulate form) | 10.00 |
| Water | 24.01 |

### Example 3

Replacing the GMO in Example 1 by 75 % glycerol monoisotearate/25 % water, or 100 % glycerol monoisostearate, or 42 % glycerol monolaurate/30 % water/28 % isostearyl alcohol, or 60 % glycerol monolaurate/40 % isostearyl alcohol produces similar results.

## Claims

1. Use of an amphiphilic organic material, capable of forming, upon contact with moisture, a water-insoluble liquid crystal phase of at least one dimensional periodicity, as an anti-sensitive teeth agent in the manufacture of a toothpaste for sensitive teeth.

2. Use according to claim 1, wherein the amphiphilic organic material is a C₁₂-C₂₄ unsaturated and/or saturated fatty acid glycerides.

3. Use according to claim 2, wherein the amphiphilic organic material is glyceryl monooleate, glyceryl monolinoleate, glyceryl monoisostearate or mixtures thereof.

4. Use according to claims 1-3, wherein the amphiphilic organic material is a material, capable of forming, upon contact with moisture, a hexagonal phase.

5. Use according to claims 1-3, wherein the amphiphilic organic material is a material, capable of forming, upon contact with moisture, a cubic phase.

6. Use according to claims 1-5, wherein the amphiphilic organic material is used in an amount of between 1 and 30% by weight of the toothpaste.

7. Use according to claim 6, wherein the amount is from 5 to 25% by weight.

8. Use according to claim 7, wherein the amount is from 5 to 15% by weight.

## Patentansprüche

1. Verwendung eines amphiphilen organischen Materials, das nach Kontakt mit Feuchtigkeit eine in Wasser unlösliche Flüssigkristallphase von mindestens eindimensionaler Periodizität bilden kann, als ein antisensitives Zahnmittel bei der Herstellung einer Zahnpasta für empfindliche Zähne.

2. Verwendung nach Anspruch 1, wobei das amphiphile organische Material ungesättigte und/oder gesättigte C₁₂-C₂₄-Fettsäureglyceride ist, bedeutet.

3. Verwendung nach Anspruch 2, wobei das amphiphile organische Material Glycerylmonooleat, Glycerylmonolinoleat, Glycerylmonoisostearat oder Gemische davon bedeutet.

4. Verwendung nach Ansprüchen 1-3, wobei das amphiphile organische Material ein Material bedeutet, das nach Kontakt mit Feuchtigkeit eine hexagonale Phase bilden kann.

5. Verwendung nach Ansprüchen 1-3, wobei das amphiphile organische Material ein Material bedeutet, das nach Kontakt mit Feuchtigkeit eine kubische Phase bilden kann.

6. Verwendung nach Ansprüchen 1-5, wobei das amphiphile organische Material in einer Menge zwischen 1 und 30 Gewichtsprozent der Zahnpasta verwendet wird.

7. Verwendung nach Anspruch 6, wobei die Menge 5 bis 25 Gewichtsprozent ist.

8. Verwendung nach Anspruch 7, wobei die Menge 5 bis 15 Gewichtsprozent ist.

## Revendications

1. Utilisation d'un matériau organique amphiphile capable, lorsque mis en contact avec de l'humidité, de former une phase cristalline liquide non soluble dans l'eau ayant une périodicité au moins à une dimension, en tant qu'agent anti-sensibilité des dents dans la fabrication d'un dentifrice pour dents sensibles.

2. Utilisation selon la revendication 1, dans laquelle le matériau amphiphile organique est des glycérides d'acides gras en C₁₂ - C₂₄ insaturés et/ou saturés.

3. Utilisation selon la revendication 2, dans laquelle le matériau organique amphiphile est du monooléate de glycéryle, du mono-linoléate de glycéryle, du mono-isostéarate de glycéryle ou des mélanges de ceux-ci.

4. Utilisation selon les revendications 1 - 3, dans laquelle le matériau organique amphiphile est un matériau capable de former une phase hexagonale lorsque mis en contact avec de l'humidité.

5. Utilisation selon les revendications 1 - 3, dans laquelle le matériau organique amphiphile est un matériau capable de former une phase cubique lorsque mis en contact avec de l'humidité.

6. Utilisation selon les revendications 1 - 5, dans laquelle le matériau organique amphiphile est utilisé dans une quantité allant de 1 à 30 % en poids de la pâte dentifrice.

7. Utilisation selon la revendication 6, dans laquelle la quantité va de 5 à 25 % en poids.

8. Utilisation selon la revendication 7, dans laquelle la quantité va de 5 à 15 % en poids.
